# EUROPEAN PATENT SPECIFICATION

(11) **EP 3 299 059 B1**
(45) Date of publication and mention of the grant of the patent: **20.10.2021**
(21) Application number: 16789602.6
(22) Date of filing: 29.04.2016
(51) Int. Cl.: A61M 39/10, A61M 5/158, A61M 39/12, A61M 5/14, A61M 25/02

(54) **JOINT DEVICE AND TRANSFUSION SET COMPRISING SAME**
GELENKVORRICHTUNG UND TRANSFUSIONSSET DAMIT
DISPOSITIF D'ASSEMBLAGE ET ENSEMBLE DE TRANSFUSION COMPRENANT CELUI-CI

(30) Priority: 01.05.2015 KR 20150062289
(43) Date of publication of application: 28.03.2018
(73) Proprietor: Sungshin Women's University Industry Academic Cooperation Foundation, Seongbuk-gu, Seoul 02844 (KR)
(72) Inventor: CHANG, Hae Jin, Seoul 06288 (KR); KIM, Mi Hyun, Suwon-si Gyeonggi-do 16530 (KR); PARK, In Hye, Gwangmyeong-si Gyeonggi-do 14271 (KR); PARK, Chan Joo, Seoul 05583 (KR); JUNG, Joo Yean, Seoul 05834 (KR)
(74) Representative: Fabry, Bernd
(86) International application number: PCT/KR2016/004559
(87) International publication number: WO 2016/178496

(56) References cited:
- WO-A1-2014/049321
- KR-A- 20160 007 012
- KR-B1- 100 529 407
- KR-B1- 100 938 880
- KR-Y1- 200 267 245
- US-A- 3 059 645
- US-A- 3 630 195
- US-A- 3 870 043
- US-A- 3 942 528
- US-A- 4 397 641
- US-A- 5 443 460
- US-A1- 2011 178 464
- US-A1- 2012 271 239

## Description

### [Technical Field]

The present invention relates to a joint device and a transfusion set comprising same, and more particularly, to a joint device connected to a syringe for administering a chemical solution included in a ringer or the like into a patient and a transfusion set comprising same.

### [Background Art]

In general, a sap solution represents a treatment method that intravenously supplies artificial solutions such as a mixture of water and an electrolyte, a carbohydrate, an amino acid solution, a glucose solution, a plasma nut, a dextran, and a red blood cell suspension or injects the artificial solution into a predetermined tissue or the artificial solution.

The transfer of the sap solution is one of general treatment methods among methods that humans and animals. Generally, the sap solution is supplied to a patient by using a transfusion set. At one end of the transfusion set, a joint device is formed which is inserted into a needle previously inserted into an arm of the patient to flow the sap.

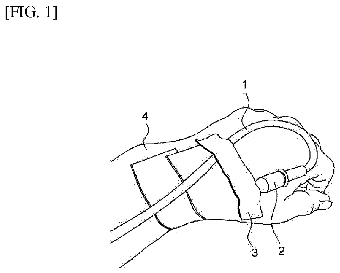

FIG. 1 is a perspective view illustrating a state in which a sap is injected by using a joint device in the related art. Referring to FIG. 1, a connection tube 1 of the transfusion set and a joint device 2 connected to the end of the connection tube 1 are illustrated. When the connection tube 1 is connected to the needle previously inserted into the arm of the patient, a part of the connection tube 1 is fixed to the arm of the patient with a band 3 or the like while drawing a large elliptical shape. This is because when the joint device 2 is straightly connected to the needle previously inserted into the arm of the patient, the patient moves or when the connection tube 1 moves in contact with surrounding objects, such a motion is just transferred to the joint device 2 and the needle connected to the end of the joint device 2 moves, and as a result, there is a problem that the patient suffers a pain.

However, even if a part of the connection tube 1 is mounted in the large elliptical shape, there is a problem that the joint device 2 may move while the connection tube 1 which still has the elliptical shape contacts the surrounding objects. Further, there is a problem that the connection tube 1 may be clogged while a part of the connection tube 1 forming the ellipse is bent. US5443460, US3942528 and US3870043 describe joint devices for transfusion sets according to the preamble of claim 1.

### [Disclosure]

### [Technical Problem]

In order to solve the problem, an object of the present invention is to provide a joint device which can reduce movement of a syringe part, which may occur due to contact of a connection tube with surrounding objects without bending and attaching a part of the connection tube in an elliptical shape and fix a part of the connection not to be bent.

The object of the present invention are not limited to the aforementioned object, and other objects, which are not mentioned above, will be apparent to a person having ordinary skill in the art from the following description.

### [Technical Solution]

In order to achieve the above object, an exemplary embodiment of the present invention provides a joint device in which one end is connected to be in communication with a needle part with a needle and the other end is connected to be in communication with a connection tube through which a solution flows, including: a body part in which both ends are opened and a chemical solution space is formed therein, and one end is connected so that the chemical solution space and the needle part are in communication with each other; and a joint part having a first end connected to be in communication with the body part, a second end connected to be in communication with the connection tube, and a communicating tube for communicating and connecting the first end and the second end with each other, in which at least a part of the communicating tube is bent so that opening directions of the first end and the second end are not opposite to each other, and the first end is rotatably joined to the body part so that the joint part relatively rotates to the body part.

In another exemplary embodiment of the present invention, the communicating tube may be extended while forming a curve from the first end toward the second end so that the opening directions of the first and second ends are the same as each other.

Here, the first end is rotatably joined to the body part so that the joint part relatively rotates to the body part.

Further, the end of the body part joined with the joint part may include a first flange formed in an inner direction of a diameter so as to prevent the first end from being separated, and the first end may include a second flange suspended on the first flange and formed to be rotatable to the body part.

In yet another exemplary embodiment of the present invention, at least a part of the body part may be made of rubber or silicon so as to be penetrated by a syringe needle of a syringe.

Here, a cross section of the chemical solution space may be formed to be larger than the cross section of the joint part so that the solution flowing in the connection tube is communicated and at the same time, the chemical solution is supplied from the syringe.

In still yet another exemplary embodiment of the present invention, the joint device may further include an extension tube interposed between the connection tube and the joint part and having a first connection end connected to be in communication with the first end and a second connection end connected to be in communication with the connection tube, in which the extension tube may have a bending part formed between the first connection end and the second connection end and bent so that the extension tube is distant on a straight line formed by the second end of the joint part and the body part.

Further, in order to achieve the above object, another exemplary embodiment of the present invention provides a transfusion set including any one of the joint devicees.

### [Effect]

According to the joint device of the present invention, the movement which occurs due to contact of the connection tube with the surrounding objects is not transferred up to the syringe needle and partially absorbed by a communicating tube to reduce the movement by the communicating tube which is at least partially bent so as to prevent an opening direction of a first end and the opening direction of a second end from being opposite to each other.

In addition, since the communicating tube is curved and extended from the first end toward the second end so that the opening directions of the first end and the second end are in the same direction, the connection tube is not mounted on the arm of the patient so as to form the large ellipse and the joint device and the connection tube may be simply connected, thereby the convenience of use be improved and the connection tube be prevented from being folded.

### [Description of Drawings]

FIG. 1 is a perspective view illustrating a state in which a sap is injected by using a joint device in the related art.
FIG. 2 is a perspective view illustrating an end component of a transfusion set including a joint device according to an exemplary embodiment of the present invention.
FIG. 3 is an exploded perspective view illustrating a state in which the joint device illustrated in FIG. 2 is disassembled.
FIG. 4 is a perspective view illustrating a state in which a sap is injected into a patient by using the transfusion set including the joint device illustrated in FIG. 2.
FIG. 5 is a plan view of the transfusion set including the joint device illustrated in FIG. 2.

### [Best Mode]

Advantages and features of the present invention, and methods for accomplishing the same will be more clearly understood from exemplary embodiments described below with reference to the accompanying drawings. However, the present invention is not limited to the following exemplary embodiments but may be implemented in various different forms. The exemplary embodiments are provided only to complete disclosure of the present invention and to fully provide a person having ordinary skill in the art to which the present invention pertains with the category of the invention, and the present invention will be defined by the appended claims.

When an element or layer is referred to as being "on" another element or layer, it may be directly on the other element or layer, or intervening elements or layers may be present. Throughout the whole specification, the same reference numerals denote the same elements.

Although the terms "first", "second", and the like are used for describing various components, these components are not confined by these terms. These terms are merely used for distinguishing one component from the other components. Therefore, a first component to be mentioned below may be a second component in a technical concept of the present invention.

A joint device of the present invention is a component used in a syringe of a component such as a transfusion set or a blood transfusion set. In the joint device, a joint part is formed to be bent. By bending the joint, it is possible to prevent a sap from being injected too quickly if the syringe is fixed to an arm of a patient. In addition, when the patient moves his/her arm, a connection tube connected with a ringer moves in contact with surrounding objects, thereby fixing a ringer needle inserted through a skin of the patient connected to the connection tube not to move.

Meanwhile, the joint device of the present invention may be used even when blood, or the like is collected from the patient and in the exemplary embodiment, the joint device will be described based on administering a ringer solution to the patient from the ringer.

Hereinafter, a joint device and a transfusion set comprising same according to the present invention will be described in detail with reference to the accompanying drawings.

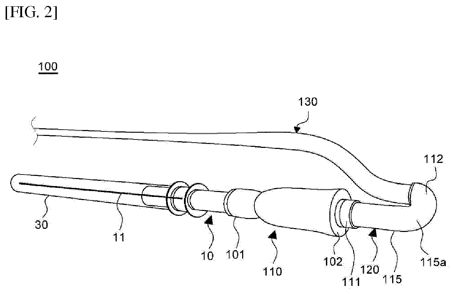

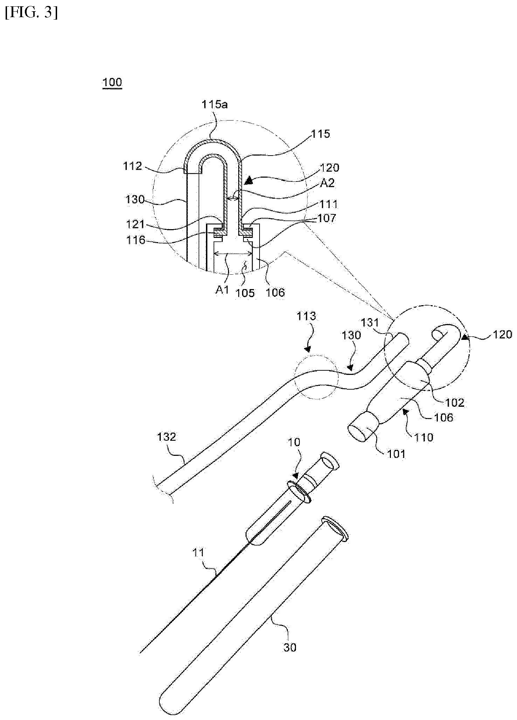

FIG. 2 is a perspective view illustrating an end component of a transfusion set including a joint device 100 according to an exemplary embodiment of the present invention and FIG. 3 is an exploded perspective view illustrating a state in which the joint device 100 illustrated in FIG. 2 is disassembled.

In the joint device 100 according to the exemplary embodiment of the present invention, one end is connected to be in communication with a needle part 10 with a needle and the other end is connected to be in communication with a connection tube (not illustrated) through which a solution such as a chemical solution is included in a ringer, and the like flows. Meanwhile, the needle part 10 may be made of a silicon material and may be configured as a partial configuration of the joint device 100 together in combination with the joint device 100. In addition, the needle part 10 may be manufactured in such a manner that an exterior of the needle part 10 is made of the silicone material, and an interior of the needle part 10 is made of a metal material and the needle part 10 is directly connected to a skin of a patient and thereafter, the interior metal material is removed.

Further, the joint device 100 according to the exemplary embodiment of the present invention includes a body part 110 and a joint part 120.

Both ends of the body part 110 are opened and a chemical solution space 105 is formed inside the body part 110. One end is connected to be in communication with the needle part 10 and the other end is connected to be in communication with the joint part 120. The chemical solution supplied from the ringer and administrated to the patient may be accommodated in the chemical solution space 105.

At least a part of the body part 110 may be made of rubber or silicon so as to be penetrated by an injection needle of a syringe. This is to allow the injection needle of the syringe to penetrate the body part 110 so that the chemical solution to be additionally administered to the patient is supplied to the chemical solution space 105. In addition, it is preferable that the chemical solution space 105 has a wider cross section than the joint part 120 so that the chemical solution additionally supplied from such the syringe is accommodated. That is, the cross section A1 of the chemical solution space 105 is preferably formed to be larger than the cross section A2 of the joint part 120 so that the chemical solution flowing in the connection tube is communicated and simultaneously the additional chemical solution is supplied from the syringe.

The joint part 120 includes a first end 111 and a second end 112 which are both ends and a communicating tube 115 which communicates and connects the first end 111 and the second end 112.

The first end 111 is connected to be in communication with the body part 110 so as to allow the chemical solution to flow. It is preferable that the joint part 120 is rotatably joined to the body part 110 through the first end 111 so that when external force is applied to the connection tube, the joint part 120 rotates relative to the body part 110 and the external force transferred to the needle part 10 is reduced.

The second end 112 is connected to be in communication with the connection tube so as to allow the chemical solution which flows out from the ringer to flow.

The communicating tube 115 is at least partially bent so that the opening direction of the first end 111 and the opening direction of the second end 112 are not opposite to each other and connects the first end 111 and the second end 112 with each other. An angle at which the communicating tube 115 is bent is preferably bent to 90 degrees or more so as to prevent the connection tube from being connected in the elliptical shape.

The opening direction of the first end 111 is toward the body part 110 and the opening direction of the second end 112 is toward the connection tube. In this case, the communicating tube 115 may be bent so that flow directions of the chemical solution flowing in the connection tube and the body part 110 are opposite to each other. In this case, the communicating tube 115 may be bent at approximately 180 degrees.

The communicating tube 115 is bent, and as a result, the force transferred to the body part 110 may be reduced even if the patient moves the arm or the connection tube hits an object. Therefore, the needle part 10 may be prevented from being moved by the force.

Further, the communicating tube 115 may be extended from the first end 111 to the second end 112 in a curved shape so that the opening directions of the first end 111 and the second end 112 are in the same direction.

The first end 111, the second end 112 and the communicating tube 115 are preferably formed as an integral tube comprising same diameter, but diameters may be different from each other by considering a velocity of the chemical solution, a curvature of the communicating tube 115, or the like.

Meanwhile, a first flange 107 may be formed to protrude on the end of the body part 110 joined with the first end 111 in an inner direction of the diameter of the body part 110 so as to prevent the first end 111 from being separated from the body part 110. In addition, a second flange 116 may be formed on the first end to protrude in an outer direction of the diameter of the body part 110 so as to be suspended to the first flange 107 and rotatable to the body part 110.

Specifically, referring to FIG. 3, the first flange 107 is formed to protrude on two locations of upper and lower portions of the second flange 116 so as to surround the second flange 116. In addition, the second flange 116 is interposed between the first flanges 107. The second flange 116 is disposed to be rotatable while being interposed between the first flanges 107. Meanwhile, a sealing member 121 may be filled between the first flange 107 and the second flange 116 so as to prevent the chemical solution from being leaked.

Meanwhile, the joint device 100 of the present invention may further include an extension tube 130.

The extension tube 130 is a tube connecting the connection tube and the joint part 120 to be in communication with each other between the connection tube connected to the ringer, or the like and the joint part 120. The extension tube 130 includes a first connection end 131 connected to be in communication with the second end 112 of the joint part 120 and a second connection end 132 connected to be in communication with the connection tube.

The needle part 10, the body part 110, and a part (the first end 111 side) of the joint part 120 are formed to be disposed arranged on a straight line at the time of joining. The extension tube 130 may further include a bending part 113 which is bent between the first connection end 131 and the second connection end 132 and is formed to be distant from the straight line formed by the second end 112 of the joint part 120 and the body part 110. The flow of the chemical solution may be controlled by the bending part 113 and the part of a ringer syringe including the joint device 100 fixed to the arm of the patient by using a band or the like and the connection tube connected from the ringer may be formed to be spatially separated from each other. Meanwhile, a joining part (not illustrated) for joining with the connection tube may be additionally formed on the second connection end 132 of the extension tube 130.

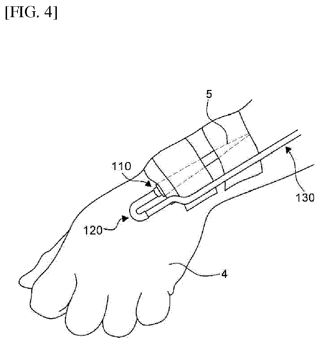

FIG. 4 is a perspective view illustrating a state in which a sap is injected into a patient by using the transfusion set including the joint device 100 illustrated in FIG. 2.

Referring to FIG. 4, a syringe 5 is inserted into an arm 4 of the patient by penetrating the skin. In addition, the body part 110 of the joint device 100 is joined to the syringe 5 and the body part 110 is fixed to the arm of the patient by the band or the like. The joint part 120 connects the body part 110 to the extension tube 130 or the connection tube.

The joint part 120 is bent in a U shape and is rigidly formed so as not to be relatively deformed by the external force so that even when the patient moves his/her arm, or the extension tube or the connection tube hits the object to move by receiving the force, the bending of the joint part 120 may reduce the movement of the syringe 5. As a result, the pain which the patient suffers due to the movement of the syringe 5 may be reduced.

Further, unlike the configuration illustrated in the drawings, the extension tube 130 or a part of the connection tube may also be fixed to the arm of the patient by a fixation member such as the band. In this case, due to the bending of the joint part 120, a therapist who fixes the transfusion set, or the like to the arm of the patient does not need to fix the end of the connection tube connected to the syringe with the band, or the like while drawing the large ellipse. In addition, it is possible to prevent the joint part 120 from being clogged due to the bending of the connection tube as the joint part 120 is already bent and rigidly formed.

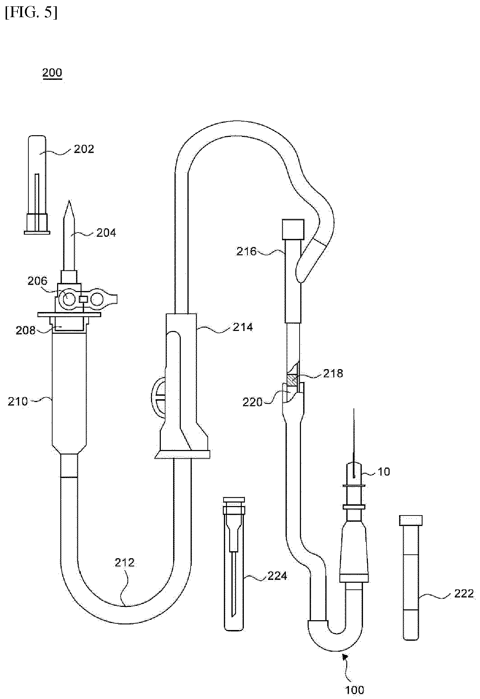

FIG. 5 is a plan view of the transfusion set 200 including the joint device 100 illustrated in FIG. 2.

The transfusion set 200 is connected from an introduction needle 204 inserted into the ringer, or the like and receiving the chemical solution to the needle part 10 connected by the joint device 100 through a connection tube 212.

Below the introduction needle 204, disposed are an air suction device 206 for sucking air, a dropping tube 208 for dropping the chemical solution, and the like, and a dropping rod 210 for forming a space in which the chemical solution passing through the dropping tube 208 stays for a predetermined time. A flow controller 214 may be disposed in the middle of the connection tube 212, which flows the flow of the chemical solution which flows in the connection tube 212 and a filter 218 filtering foreign materials included in the chemical solution and a linkage tube 220 forming a tube so as to allow the chemical solution passing through the filter 218 to flow to the joint device 100 may be disposed close to the joint device 100. Meanwhile, a chemical solution injection unit 216 may be additionally disposed, which may additionally inject the chemical solution through the syringe. In addition, protective covers 202 and 222 may be provided to cover the introduction needle 204 and the needle part 10, respectively.

By the transfusion set 200, when the needle part 10 is mounted on the arm of the patient by the bent tube of the joint device 100 or the joint device 100 is connected to the needle part 10 which is already mounted, the connection tube 212 need not be bent and formed in a long elliptical shape.

Hereinabove, the exemplary embodiments of the present invention have been described with the accompanying drawings, but it can be understood by those skilled in the art that the present invention can be executed in other detailed forms without changing the requisite features of the present invention. Therefore, it should be appreciated that the aforementioned exemplary embodiments are illustrative in all aspects and are not restricted.

## Claims

1. A joint device (100) for a transfusion set in which one end is connected to be in communication with a needle part (10) with a needle and the other end is connected to be in communication with a connection tube (212) through which a solution flows, the joint device (100) comprising:
a body part (110) in which both ends are opened and a chemical solution space (105) is formed therein, and one end is connected so that the chemical solution space (105) and the needle part (10) are in communication with each other; and
a joint part (120) having a first end (111) connected to be in communication with the body part (110), a second end (112) connected to be in communication with the connection tube (212), and a communicating tube (115) for communicating and connecting the first end (111) and the second end (112) with each other,
wherein at least a part of the communicating tube (115) is bent so that opening directions of the first end (111) and the second end (112) are not opposite to each other,
**characterized in that** the first end (111) is rotatably joined to the body part (110) so that the joint part (120) relatively rotates to the body part (110).

2. The joint device (100) of claim 1, wherein the communicating tube (115) is extended while forming a curve from the first end (111) toward the second end (112) so that the opening directions of the first and second ends (111,112) are the same as each other.

3. The joint device (100) of claim 2, wherein the end of the body part (110) joined with the joint part (120) includes
a first flange (107) formed in an inner direction of a diameter so as to prevent the first end (111) from being separated,
and the first end (111) includes a second flange (116) suspended on the first flange (107) and formed to be rotatable to the body part (110).

4. The joint device (100) of claim 1, wherein at least a part of the body part (110) is made of rubber or silicon so as to be penetrated by a syringe needle of a syringe (5).

5. The joint device (100) of claim 4, wherein a cross section (A1) of the chemical solution space (105) is formed to be larger than the cross section (A2) of the joint part (120) so that the solution flowing in the connection tube (212) is communicated and at the same time, the chemical solution is supplied from the syringe (5).

6. The joint device (100) of claim 1, further comprising:
an extension tube (130) interposed between the connection tube (212) and the joint part (120) and having a first connection end (131) connected to be in communication with the first end (111) and a second connection end (132) connected to be in communication with the connection tube (212),
wherein the extension tube (130) has a bending part (113) formed between the first connection end (131) and the second connection end (132) and bent so that the extension tube (130) is distant on a straight line formed by the second end (112) of the joint part (120) and the body part (110).

7. A transfusion set including the joint device of any one of claims 1 to 6.

## Patentansprüche

1. Eine Verbindungsvorrichtung (100) für ein Transfusionsset, bei dem ein Ende so verbunden ist, dass es mit einem Nadelteil (10) mit einer Nadel in Verbindung steht, und das andere Ende so verbunden ist, dass es mit einem Verbindungsrohr (212) in Verbindung steht, durch das eine Lösung fließt, wobei die Verbindungsvorrichtung (100) umfasst:
ein Körperteil (110), in dem beide Enden geöffnet sind und in dem ein Raum (105) für eine chemische Lösung ausgebildet ist, und ein Ende so verbunden ist, dass der Raum (105) für die chemische Lösung und das Nadelteil (10) miteinander in Verbindung stehen; und
ein Verbindungsteil (120) mit einem ersten Ende (111), das so verbunden ist, dass es mit dem Körperteil (110) in Verbindung steht, einem zweiten Ende (112), das so verbunden ist, dass es mit dem Verbindungsrohr (212) in Verbindung steht, und einem Verbindungsrohr (115) zum Kommunizieren und Verbinden des ersten Endes (111) und des zweiten Endes (112) miteinander,
wobei zumindest ein Teil des Verbindungsrohrs (115) so gebogen ist, dass die Öffnungsrichtungen des ersten Endes (111) und des zweiten Endes (112) nicht entgegengesetzt zueinander sind,
**dadurch gekennzeichnet, dass** das erste Ende (111) drehbar mit dem Körperteil (110) verbunden ist, so dass das Gelenkteil (120) relativ zu dem Körperteil (110) rotiert.

2. Die Verbindungsvorrichtung (100) nach Anspruch 1, wobei sich das Verbindungsrohr (115) unter Bildung einer Kurve vom ersten Ende (111) zum zweiten Ende (112) erstreckt, so dass die Öffnungsrichtungen des ersten und des zweiten Endes (111, 112) einander entsprechen.

3. Die Verbindungsvorrichtung (100) nach Anspruch 2, wobei das Ende des Körperteils (110), das mit dem Verbindungsteil (120) verbunden ist, folgendes aufweist
einen ersten Flansch (107), der in einer inneren Richtung eines Durchmessers ausgebildet ist, um zu verhindern, dass das erste Ende (111) getrennt wird, und
das erste Ende (111) einen zweiten Flansch (116) aufweist, der an dem ersten Flansch (107) aufgehängt und so ausgebildet ist, dass er an dem Körperteil (110) drehbar ist.

4. Die Verbindungsvorrichtung (100) nach Anspruch 1, wobei mindestens ein Teil des Körperteils (110) aus Gummi oder Silikon hergestellt ist, so dass er von einer Spritzennadel einer Spritze (5) durchstochen werden kann.

5. Die Verbindungsvorrichtung (100) nach Anspruch 4, wobei ein Querschnitt (A1) des Raums für die chemische Lösung (105) größer ausgebildet ist als der Querschnitt (A2) des Verbindungsteils (120), so dass die Lösung, die in dem Verbindungsrohr (212) fließt, kommuniziert wird und gleichzeitig die chemische Lösung aus der Spritze (5) zugeführt wird.

6. Die Verbindungsvorrichtung (100) nach Anspruch 1 ferner umfassend:
ein Verlängerungsrohr (130), das zwischen dem Verbindungsrohr (212) und dem Verbindungsteil (120) angeordnet ist und ein erstes Verbindungsende (131) aufweist, das so verbunden ist, dass es mit dem ersten Ende (111) in Verbindung steht, und ein zweites Verbindungsende (132), das so verbunden ist, dass es mit dem Verbindungsrohr (212) in Verbindung steht,
wobei das Verlängerungsrohr (130) einen Biegeteil (113) aufweist, der zwischen dem ersten Verbindungsende (131) und dem zweiten Verbindungsende (132) ausgebildet ist und so gebogen ist, dass das Verlängerungsrohr (130) von einer geraden Linie entfernt ist, die durch das zweite Ende (112) des Verbindungsteils (120) und des Körperteils (110) gebildet wird.

7. Ein Transfusionsset enthaltend die Verbindungsvorrichtung nach einem der Ansprüche 1 bis 6.

## Revendications

1. Dispositif de raccordement (100) pour un ensemble de transfusion dans lequel une extrémité est raccordée pour être en communication avec une partie aiguille (10) dotée d'une aiguille et l'autre extrémité est raccordée pour être en communication avec un tube de raccordement (212) à travers lequel s'écoule une solution, le dispositif de raccordement (100) comprenant :
une partie corps (110) dans laquelle les deux extrémités sont ouvertes et au sein de laquelle est formé un espace de solution chimique (105), et une extrémité est raccordée de sorte que l'espace de solution chimique (105) et la partie aiguille (10) soient en communication l'un avec l'autre ; et
une partie de raccordement (120) possédant une première extrémité (111) raccordée pour être en communication avec la partie corps (110), une seconde extrémité (112) raccordée pour être en communication avec le tube de raccordement (212), et un tube de communication (115) pour faire communiquer et raccorder la première extrémité (111) et la seconde extrémité (112) entre elles,
dans lequel au moins une partie du tube de communication (115) est courbée de sorte que des directions d'ouverture de la première extrémité (111) et de la seconde extrémité (112) ne soient pas opposées l'une à l'autre,
**caractérisé en ce que** la première extrémité (111) est raccordée de manière rotative à la partie corps (110) de sorte que la partie de raccordement (120) tourne relativement à la partie corps (110).

2. Dispositif de raccordement (100) selon la revendication 1, dans lequel le tube de communication (115) est étendu tout en formant une courbe depuis la première extrémité (111) en direction de la seconde extrémité (112) de sorte que les directions d'ouverture des première et seconde extrémités (111, 112) soient identiques entre elles.

3. Dispositif de raccordement (100) selon la revendication 2, dans lequel l'extrémité de la partie corps (110) raccordée avec la partie de raccordement (120) comprend
une première bride (107) formée dans une direction interne d'un diamètre de façon à empêcher que la première extrémité (111) soit séparée,
et la première extrémité (111) comprend une seconde bride (116) suspendue sur la première bride (107) et formée pour pouvoir tourner par rapport à la partie corps (110).

4. Dispositif de raccordement (100) selon la revendication 1, dans lequel au moins une partie de la partie corps (110) est en caoutchouc ou silicium de façon à être pénétrée par une aiguille de seringue d'une seringue (5).

5. Dispositif de raccordement (100) selon la revendication 4, dans lequel une coupe transversale (A1) de l'espace de solution chimique (105) est formée pour être plus grande qu'une coupe transversale (A2) de la partie de raccordement (120) de sorte que la solution s'écoulant dans le tube de raccordement (212) soit communiquée et, en même temps, que la solution chimique soit fournie depuis la seringue (5).

6. Dispositif de raccordement (100) selon la revendication 1, comprenant en outre :
un tube d'extension (130) interposé entre le tube de raccordement (212) et la partie de raccordement (120) et possédant une première extrémité de raccordement (131) raccordée pour être en communication avec la première extrémité (111) et une seconde extrémité de raccordement (132) raccordée pour être en communication avec le tube de raccordement (212),
dans lequel le tube d'extension (130) possède une partie de courbure (113) formée entre la première extrémité de raccordement (131) et la seconde extrémité de raccordement (132) et courbée de sorte que le tube d'extension (130) soit distant sur une ligne droite formée par la seconde extrémité (112) de la partie de raccordement (120) et la partie corps (110).

7. Ensemble de transfusion comprenant le dispositif de raccordement selon l'une quelconque des revendications 1 à 6.
